# EUROPEAN PATENT APPLICATION

(11) **EP 3 581 270 A1**
(43) Date of publication of application: **18.12.2019**
(21) Application number: 18750787.6
(22) Date of filing: 09.02.2018
(51) Int. Cl.: B01J 35/02, A61L 9/00, B01J 21/06, B01J 35/06, B01J 37/02, B01J 37/34, F24F 7/00

(54) **PHOTOCATALYST-CARRYING MESH SHEET, AIR PURIFIER, AND METHOD FOR PRODUCING PHOTOCATALYST-CARRYING MESH SHEET**

(30) Priority: 13.02.2017 JP 2017023802
(71) Applicant: Sunstar Engineering Inc., Osaka 569-1134 (JP); U-VIX Corporation, Tokyo 152-0034 (JP)
(72) Inventor: KIMURA, Yoshiki, Takatsuki-shi Osaka 569-1134 (JP); YAMAGUCHI, Katsuhiro, Takatsuki-shi Osaka 569-1134 (JP); OKA, Toru, Takatsuki-shi Osaka 569-1134 (JP); MORITO, Yuko, Tokyo 152-0034 (JP)
(74) Representative: TBK
(86) International application number: PCT/JP2018/004723
(87) International publication number: WO 2018/147444

(57) **Abstract**

It is an object of the present invention to provide a photocatalyst-carrying mesh sheet which firmly supports anatase type titanium oxide as a photocatalyst to prevent peeling, and increases opportunities for contact with the photocatalyst to significantly improve a purification treatment efficiency provided by the photocatalyst and to suppress manufacturing cost. A photocatalyst-carrying mesh sheet (S1) is disclosed, which includes: a net-form titanium sheet (11) having a periodic pattern; a titanium oxide film (3) formed on a surface of the net-form titanium sheet (11); and an anatase type titanium oxide particle (4) supported on the titanium oxide film (3).

## Description

The present application is an entrance into U.S from PCT/JP2018/004723 filed on February 9, 2018 which claims a benefit of priority to Japanese patent application No. 2017-023802 filed on February 13, 2017.

### Technical Field

The present invention relates to a photocatalyst-carrying mesh sheet, an air purifier, and a method for producing a photocatalyst-carrying mesh sheet.

### Background Art

Anatase type titanium oxide is known as a photocatalyst. When the anatase type titanium oxide is irradiated with ultraviolet light, the anatase type titanium oxide produces holes to generate active species such as hydroxy radicals (·OH). This causes an organic material to be decomposed, to provide a deodorizing effect and a sterilization effect, and therefore, the anatase type titanium oxide is applied to an air purifier and the like.

For example, Japanese Patent No. 5474612 is known as anatase type titanium oxide used as a photocatalyst. Japanese Patent No. 5474612 discloses a photocatalyst sheet. In the photocatalyst sheet, a titanium oxide base as an anode oxide film is formed on the surface of a titanium foil having an aperiodic sponge structure having a large number of fine flow paths penetrating the titanium foil. The fine flow paths are formed by subjecting one surface or both surfaces to an etching treatment depending on an aperiodic pattern. Anatase type titanium oxide particles are baked on the titanium oxide base.

This makes it possible to firmly support the anatase type titanium oxide as the photocatalyst, to prevent peeling, and to increase opportunities for contact with the photocatalyst to significantly improve a purification treatment efficiency provided by the photocatalyst. However, in Japanese Patent No. 5474612, the titanium foil is subjected to the etching treatment, which concernedly causes high manufacturing cost, whereby a further improvement such as cost suppression is required.

Meanwhile, Japanese Patent Application Laid-Open No. 2005-254167 is known as an example using another metal. Japanese Patent Application Laid-Open No. 2005-254167 discloses a photocatalyst supporting metal filter. The photocatalyst supporting metal filter includes a metal filter substrate and a photocatalyst layer formed on the metal filter substrate. In the metal filter substrate, a metal surface has a plurality of opening parts formed so that the opening ratio of the opening parts is 30% or more and 90% or less with respect to the metal surface, and a remaining part other than the opening parts in the metal surface is subjected to a roughening processing.

However, the use of the anatase type titanium oxide and a metal substrate other than titanium causes lowered activity of the anatase type titanium oxide, so that a purification treatment efficiency provided by the photocatalyst is disadvantageously lowered.

### Disclosure of Invention

### Problems to be Solved by the Invention

Therefore, it is an object of the present invention to provide a photocatalyst-carrying mesh sheet which firmly supports anatase type titanium oxide as a photocatalyst to prevent peeling, and increases opportunities for contact with the photocatalyst to significantly improve a purification treatment efficiency provided by the photocatalyst and to suppress manufacturing cost.

### Means for Solving the Problem

In order to solve the above problems, a photocatalyst-carrying mesh sheet of the present invention includes: a net-form titanium sheet having a periodic pattern; a titanium oxide film formed on a surface of the net-form titanium sheet; and anatase type titanium oxide particles supported on the titanium oxide film.

### Effect of invention

The present invention can provide a photocatalyst-carrying mesh sheet which firmly supports anatase type titanium oxide as a photocatalyst, and increases opportunities for contact with the photocatalyst to significantly improve a purification treatment efficiency provided by the photocatalyst and to suppress manufacturing cost.

### Brief Description of Drawings

FIG. 1 is a schematic view showing an example of a photocatalyst-carrying mesh sheet according to the present invention.
FIG. 2 is a schematic view showing an example of a method for producing the photocatalyst-carrying mesh sheet of FIG. 1.
FIG. 3 is a schematic view showing another example of the photocatalyst-carrying mesh sheet according to the present invention.
FIG. 4 is a schematic view showing an example of an air purifier including the photocatalyst-carrying mesh sheet according to the present invention.
FIG. 5 is a graph showing the evaluation results of Example 1.

### Best Mode for Carrying Out the Invention

Hereinafter, a photocatalyst-carrying mesh sheet, an air purifier, and a method for producing a photocatalyst-carrying mesh sheet according to the present invention will be described with reference to the drawings. The present invention is not limited to the embodiments described below, but changes such as other embodiments, addition, modification, and deletion are available within the scope of conception of a person skilled in the art. Any mode is included within the scope of the present invention as long as the mode exhibits the working and effect of the present invention.

A photocatalyst-carrying mesh sheet of the present invention includes: a net-form titanium sheet having a periodic pattern; a titanium oxide film formed on a surface of the net-form titanium sheet; and anatase type titanium oxide particles supported on the titanium oxide film.

A method for producing a photocatalyst-carrying mesh sheet of the present invention includes the steps of: periodically forming a plurality of flaws in a metal substrate made of titanium, and pulling the metal substrate to form an expanded titanium mesh sheet having a net-form and a periodic pattern; subjecting the expanded titanium mesh sheet to an anode oxidation treatment and/or a heat treatment to form a titanium oxide film on a surface of the expanded titanium mesh sheet; and supporting anatase type titanium oxide particles on the titanium oxide film.

A method for producing a photocatalyst-carrying mesh sheet of the present invention includes the steps of: weaving a titanium wire into a titanium wire mesh sheet having a net-form and a periodic pattern; subjecting the titanium wire mesh sheet to an anode oxidation treatment and/or a heat treatment to form a titanium oxide film on a surface of the titanium wire mesh sheet; and supporting anatase type titanium oxide particles on the titanium oxide film.

### (First Embodiment)

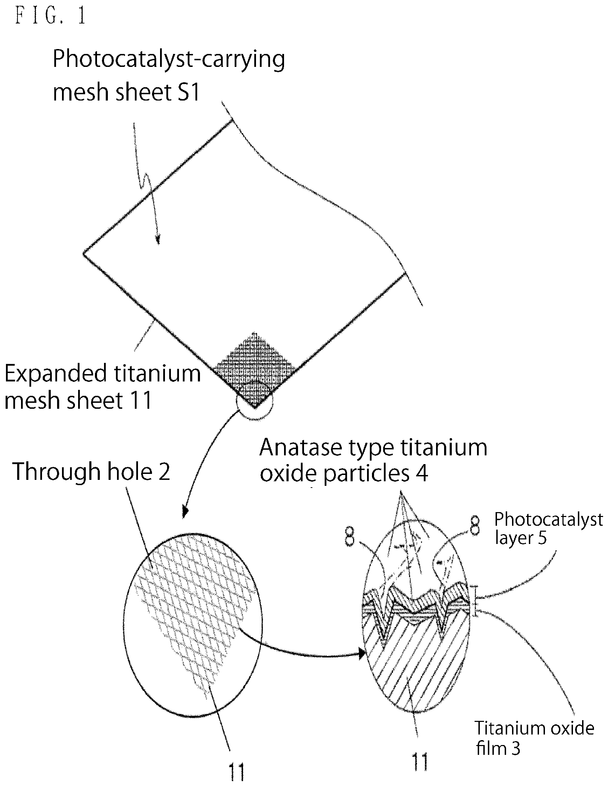

FIG. 1 shows a schematic view of an embodiment of a photocatalyst-carrying mesh sheet according to the present invention. In FIG. 1, a part of the photocatalyst-carrying mesh sheet of the present embodiment is shown, and an enlarged schematic view and a cross-sectional schematic view thereof are shown.

In a photocatalyst-carrying mesh sheet S1 of the present embodiment, a titanium oxide film 3 is formed on the surface of an expanded titanium mesh sheet 11, and anatase type titanium oxide particles 4 are supported on the titanium oxide film 3 to form a photocatalyst layer 5.

The expanded titanium mesh sheet 11 is a net-form titanium sheet having a periodic pattern, and is obtained by expanding a titanium sheet. In the present embodiment, the expanding method can be appropriately changed, but a plurality of flaws are formed in the titanium sheet, and both ends of the titanium sheet are extended to produce a net-form titanium sheet having a periodic pattern.

The plurality of flaws may or may not penetrate the titanium sheet.

A method for forming the flaws can be appropriately changed, and examples thereof include a method for forming flaws according to laser irradiation, a method for forming flaws according to pressing, and a method for forming flaws using an ultrasonic cutter. Among them, the flaws are preferably formed by pressing or laser irradiation.

The plurality of flaws are preferably formed at intervals of, for example, 0.05 to 3 mm. By pulling the titanium sheet in which the plurality of flaws are formed, a plurality of through holes 2 as shown in FIG. 1 are periodically formed.

The method for forming the plurality of flaws can be appropriately changed, and it is preferable that the flaws are diagonally formed in the titanium sheet to make holes penetrate. In this case, when an expanded metal is provided by pulling, holes are formed so as to laterally expand to provide increased air resistance. This provides an increased contact area for air purification to provide a further improved purification efficiency.

The thickness of the expanded titanium mesh sheet 11 is preferably, for example, 0.05 to 2 mm.

The length of an opening part of the expanded titanium mesh sheet 11 in a short width direction is preferably 0.05 to 5 mm, and more preferably 0.05 to 1.5 mm. The length of the opening part of the expanded titanium mesh sheet 11 in a long width direction is preferably 0.1 to 10 mm, and more preferably 0.1 to 3 mm. The open ratio of the expanded titanium mesh sheet 11 is preferably 5 to 95%, and more preferably 10 to 80%.

In this case, the flowability of air in the obtained photocatalyst-carrying mesh sheet is improved, to increase opportunities for contact with a photocatalyst to further improve a purification treatment efficiency provided by the photocatalyst. The density of the expanded titanium mesh sheet is increased, to maintain desired strength of the expanded titanium mesh sheet, whereby a desired shape of the expanded titanium mesh sheet is likely to be maintained even when the expanded titanium mesh sheet is used for a member such as an air purifier.

The size of the opening part of the expanded titanium mesh sheet 11 is substantially the same as the size of the opening part of the obtained photocatalyst supporting sheet.

The expanded titanium mesh sheet 11 is a net-form titanium sheet having a periodic pattern, but the expanded titanium mesh sheet 11 does not necessarily have a periodic pattern in all places, preferably 50% or more of the places, and more preferably 80% of the places.

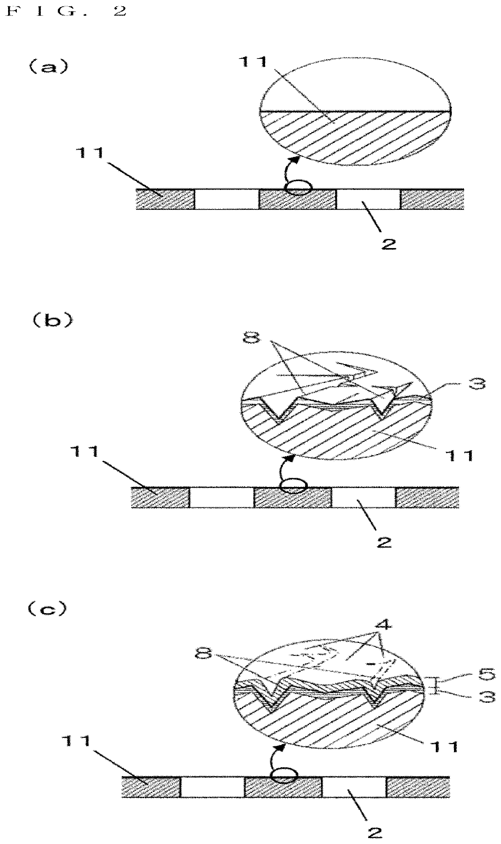

A diagram for illustrating an example of a method for producing the photocatalyst supporting sheet in the present embodiment is shown in FIG. 2. FIG. 2 shows a part of a manufacturing process, and the manufacturing process of the expanded titanium mesh sheet 11 is omitted.

FIG. 2A shows a cross-sectional view of the expanded titanium mesh sheet 11 and a partially enlarged view thereof. First, expanded titanium mesh sheet 11 is subjected to an anode oxidation treatment to form the titanium oxide film 3 on the surface of the expanded titanium mesh sheet 11. The anode oxidation treatment is performed by applying a predetermined voltage between the expanded titanium mesh sheet 11 serving as an anode and a cathode in a phosphoric acid bath (for example, an aqueous solution containing 3% phosphoric acid). As a result, as shown in FIG. 2B, the surface of the expanded titanium mesh sheet 11 is oxidized to form an anode oxide film (titanium oxide film 3).

At this time, the oxide film is formed not only on both the front and back surfaces of the expanded titanium mesh sheet 11 but also on the entire surface exposed to the phosphoric acid bath such as the inner wall surface of a through hole 2.

Thereafter, the expanded titanium mesh sheet 11 on which the oxide film is formed is subjected to a heat treatment. The heating is performed, for example, in the atmosphere at 450 to 550°C for 2 to 3 hours. By the heat treatment, the anode oxide film is less likely to be peeled off. When the surface is enlarged and observed, cracks 8 caused by the anode oxidation treatment and/or the heat treatment appear.

It is known that, when the anode oxidation treatment is performed, different colors are developed due to light interference depending on the thickness of the oxide film, and the oxide film exhibits a purple color at a thickness of about 70 nm, a green color at about 150 nm, and a pink color at about 200 nm. In the present embodiment, a film having a thickness of 70 to 150 nm is used.

The expanded titanium mesh sheet 11 may be subjected to a roughening processing before the anode oxidation treatment and/or the heat treatment. In this case, the oxide film can be less likely to be peeled off. The roughening processing can be appropriately changed, and examples of the roughening processing include lapping processing.

Next, a baking treatment is performed to support the anatase type titanium oxide particles 4.

When the expanded titanium mesh sheet 11 having the titanium oxide film 3 formed on the surface thereof is dipped in a slurry in which the anatase type titanium oxide particles 4 are dispersed, and then baked at 400 to 550°C, the photocatalyst layer 5 is formed on both the front and back surfaces of the expanded titanium mesh sheet 11 and the inner wall surface of the through hole 2, as shown in FIG. 2C.

The titanium oxide of the titanium oxide film 3 and the titanium oxide of the photocatalyst layer 5 are bonded to each other, whereby the bonding property is extremely improved, and as a result, the photocatalyst layer 5 is less likely to be peeled off.

As a method for supporting the anatase type titanium oxide particles 4, for example, spray coating and the like may be performed in addition to the dipping described above. In the spray coating, the slurry in which the anatase type titanium oxide particles 4 are dispersed can be spray-applied, and then baked to form the photocatalyst layer 5.

It is preferable that the anatase type titanium oxide particles 4 are supported, and a step of sending a jet wind is then performed as necessary. By this step, the titanium oxide particles adhering to the surface and apt to be peeled off can be removed. Thus, the photocatalyst-carrying mesh sheet S1 of the present embodiment is obtained.

The opening ratio of the photocatalyst-carrying mesh sheet S1 of the present embodiment obtained as described above is preferably 5 to 95%, and more preferably 10 to 80%. In this case, the flowability of air in the obtained photocatalyst-carrying mesh sheet is improved, to increase opportunities for contact with a photocatalyst to further improve a purification treatment efficiency provided by the photocatalyst.

As described above, according to the present embodiment, the titanium oxide of the titanium oxide film 3 and the titanium oxide of the photocatalyst layer 5 are bonded to each other, whereby the anatase type titanium oxide as a photocatalyst can be firmly supported to prevent peeling.

By using the net-form titanium sheet (expanded titanium mesh sheet 11) having a periodic pattern, the surface area is increased, which provides a remarkably improved treatment efficiency. Therefore, the opportunities for contact with the photocatalyst can be increased, to remarkably improve a purification treatment efficiency provided by the photocatalyst. The net-form titanium sheet is used, whereby a natural air flow can provide the purification efficiency due to the photocatalyst without providing a fan and the like to create an air flow.

Furthermore, the titanium oxide film 3 made of the anode oxide film has the fine cracks 8 of micron order, whereby not only the photocatalyst layer 5 is strongly bonded thereon, but also the surface area is increased, which provides a remarkably improved treatment efficiency. Upon irradiation with UV light, irregular reflection/light scattering occurs on the surface of the photocatalyst layer 5 and the interface with the titanium oxide film 3, whereby the UV light can be efficiently used.

By using the expanded titanium mesh sheet 11, the manufacturing cost, particularly the cost of etching can be suppressed as compared with conventional one in which titanium is etched (for example, Japanese Patent No. 5474612). Since the photocatalyst-carrying mesh sheet S1 has excellent heat resistance and chemical resistance, the photocatalyst-carrying mesh sheet S1 can be used under severe use conditions.

Furthermore, since the photocatalyst-carrying mesh sheet S1 is formed in a sheet shape, the irradiation of both the surfaces of the photocatalyst-carrying mesh sheet S1 can be allowed depending on the arrangement of a light source, and the photocatalyst-carrying mesh sheet S1 can be multilayered. In this case, a further improvement in a photocatalytic effect can also be expected.

### (Second Embodiment)

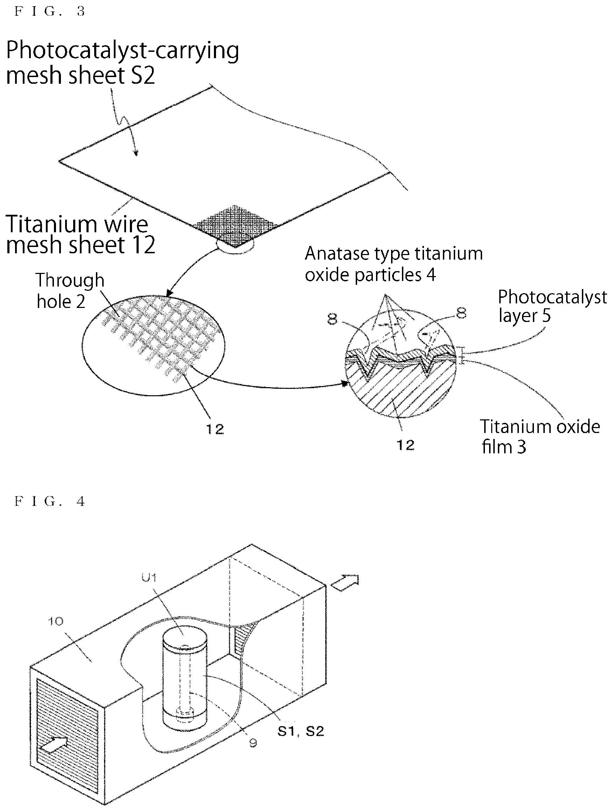

FIG. 3 shows a schematic view of another embodiment of a photocatalyst-carrying mesh sheet according to the present invention. Descriptions may be omitted for the matters common to the above embodiment. In FIG. 3, a part of the photocatalyst-carrying mesh sheet of the present embodiment is shown, and an enlarged schematic view and a cross-sectional schematic view thereof are shown.

In a photocatalyst-carrying mesh sheet S2 of the present embodiment, a titanium oxide film 3 is formed on the surface of a titanium wire mesh sheet 12, and anatase type titanium oxide particles 4 are supported on the titanium oxide film 3 to form a photocatalyst layer 5.

Since the titanium wire mesh sheet 12 is a net-form titanium sheet having a periodic pattern, as with an expanded titanium mesh sheet 11, the surface area is increased, which provides a remarkably improved treatment efficiency. Therefore, the opportunities for contact with the photocatalyst can be increased, to remarkably improve a purification treatment efficiency provided by the photocatalyst.

By using the titanium wire mesh sheet 12, the manufacturing cost, particularly the cost of etching can be suppressed as compared with conventional one in which titanium is etched (for example, Japanese Patent No. 5474612). Since the photocatalyst-carrying mesh sheet S2 has excellent heat resistance and chemical resistance, the photocatalyst-carrying mesh sheet S2 can be used under severe use conditions.

The titanium wire mesh sheet 12 is obtained by weaving a titanium wire. An example in the case of the plain weave of the titanium wire mesh sheet 12 is shown in FIG. 3, but the titanium wire mesh sheet 12 is not limited thereto, and the other examples include twill weave, tatami weave, plain tatami weave, and twill tatami weave.

The opening ratio of the titanium wire mesh sheet 12 is preferably 5 to 95%, and more preferably 10 to 80%. In this case, the flowability of air in the obtained photocatalyst-carrying mesh sheet is improved, to increase opportunities for contact with a photocatalyst to further improve a purification treatment efficiency provided by the photocatalyst. The density of the titanium wire mesh sheet is increased, to maintain desired strength of the titanium wire mesh sheet, whereby a desired shape of the titanium wire mesh sheet is likely to be maintained even when the titanium wire mesh sheet is used for a member such as an air purifier.

The size of the opening part of the titanium wire mesh sheet 12 is substantially the same as the size of the opening part of the obtained photocatalyst supporting sheet.

The diameter of a titanium wire is preferably 50 to 5000 µm.

The thickness of the titanium wire mesh sheet 12 is preferably 0.05 to 3 mm.

The titanium wire mesh sheet 12 is a net-form titanium sheet having a periodic pattern, but the titanium wire mesh sheet 12 does not necessarily have a periodic pattern in all places, preferably 50% or more of the places, and more preferably 80% or more of the places.

### (Third Embodiment)

When a titanium oxide film is formed on the surfaces of an expanded titanium mesh sheet 11 and titanium mesh sheet 12, the surfaces may be oxidized by supplying air and performing a heat treatment without performing anode oxidation, followed by forming the oxide film on the surfaces of expanded titanium mesh sheet 11 and titanium mesh sheet 12. Descriptions of matters common to the first and second embodiments will be omitted.

First, in order to form the titanium oxide film on the surfaces of the expanded titanium mesh sheet 11 and the titanium mesh sheet 12, heat oxidation is performed in the atmosphere.

The heating is performed, for example, in the atmosphere at 400 to 750°C for 30 minutes to 3 hours. By subjecting the surface to the heat oxidation treatment, the dense oxide film and cracks 8 caused by the heat oxidation treatment appear when the surface is enlarged and observed.

By oxidizing the expanded titanium mesh sheet 11 and the titanium mesh sheet 12 while flowing air, the oxide film can be formed not only on the front and back surfaces of the expanded titanium mesh sheet 11 and the titanium mesh sheet 12 but also on the entire surface such as the inner wall surface of a through hole 2.

In the case of an atmospheric oxidation treatment, the thickness of the oxide film to be formed varies depending on the temperature and time. It is known that the film thickness is about 20 to 40 nm at 500°C, about 100 nm at 600°C, and about 130 nm at 700°C. It is known that as the film thickness is more, the corrosion resistance is improved.

In the present embodiment, a film having a thickness of 20 to 150 nm is used.

The oxide film formed by the heat treatment has higher adhesion than that of the film formed by the anode oxidation shown in the first and second embodiments, which makes it possible to support a more photocatalyst.

### (Fourth Embodiment)

Next, an example of an air purifier including a photocatalyst-carrying mesh sheet of the present invention will be described. As shown in FIG. 4, photocatalyst-carrying mesh sheets S1 and S2 of the above embodiment are cylindrically wound, and a photocatalyst unit U1 having an ultraviolet light source 9 disposed at the center thereof is provided. This can be used so as to be exposed to an air flow flowing in a treatment chamber 10 of the air purifier.

The photocatalyst-carrying mesh sheet of the present invention is flexible, and can be used in various shapes such as a cylindrical shape, a spherical shape, a bellows shape, and a funnel shape. The photocatalyst-carrying mesh sheet can be bent into any shape depending on the specifications of the air purifier, or wound.

### Examples

Hereinafter, the present invention will be described in detail by way of Examples, but the present invention is not limited to the following Examples.

### (Example 1)

A titanium sheet was pressed by using a roll pressing machine manufactured by Sunk Metal Co., Ltd., to form a plurality of flaws penetrating the titanium sheet at intervals of 0.1 mm. By pulling the titanium sheet, an expanded titanium mesh sheet having a periodic pattern was obtained (thickness: 0.2 mm). At this time, the opening ratio of the obtained expanded titanium mesh sheet was 48%. The length of an opening part of the expanded titanium mesh sheet in a short width direction was 0.3 mm, and the length of the opening part in a long width direction was 0.7 mm.

Next, an anode oxidation treatment was performed to form a titanium oxide film as follows. The anode oxidation treatment was performed by applying a predetermined voltage between the expanded titanium mesh sheet serving as an anode and a cathode in a phosphoric acid bath (aqueous solution containing 3% phosphoric acid), and the surface of the expanded titanium mesh sheet was oxidized to form an anode oxide film.

Next, the expanded titanium mesh sheet on which the oxide film was formed was subjected to a heat treatment. The heating was performed in the atmosphere at 450°C for 2 hours. By performing the heat treatment, the titanium oxide film in which the anode oxide film was heated was formed. In the present Example, a film having a thickness of 70 to 150 nm was formed.

The expanded titanium mesh sheet having the titanium oxide film formed on the surface was dipped in a slurry in which anatase type titanium oxide particles were dispersed, and then baked at 400 to 450°C, to support the anatase type titanium oxide particles, thereby forming a photocatalyst layer. Thereafter, a step of sending a jet wind was performed to remove the easily peelable titanium oxide particles adhering to the surface. Thereby, a photocatalyst-carrying mesh sheet of the present Example was obtained. The opening ratio of the photocatalyst-carrying mesh sheet obtained in the present Example was 47%.

Next, the photocatalyst-carrying mesh sheet obtained in the present Example was subjected to the following evaluation. As the evaluation, an air purifier as shown in FIG. 4 was placed in a closed space having a volume of 1 m³, and the concentration change of acetaldehyde having a predetermined concentration in the closed space was measured with time while a wind having a wind speed of 5.5 m/s was blown against the air purifier. As the air purifier, a photocatalyst unit U1 in which an A5 size photocatalyst-carrying mesh sheet S1 was doubly wound was placed, and an ultraviolet light source 9 having a wavelength of 254 nm was disposed at the center so as to be exposed to an air flow flowing in a treatment chamber 10.

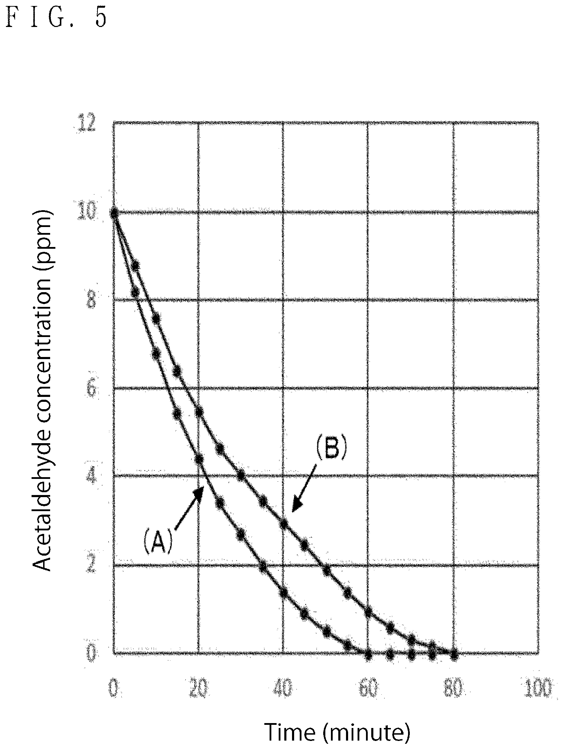

The results are shown in FIG. 5. In FIG. 5, the results of the photocatalyst-carrying mesh sheet obtained in the present Example are shown by (A) in FIG. 5. (B) in FIG. 5 is a graph of the photocatalyst sheet of Japanese Patent No. 5474612 subjected to the same measurement as the above. In (B) in FIG. 5, a titanium foil is etched with a non-periodic pattern to form an aperiodic sponge structure, and the anatase type titanium oxide particles are supported on the surface.

As shown in FIG. 5, it is found that the photocatalyst-carrying mesh sheet obtained in the present Example provides a decreased concentration (ppm) of acetaldehyde gas with the passage of time, to provide good deodorizing performance. Good results are obtained even in the example shown by (B) in FIG. 5, but the photocatalyst-carrying mesh sheet obtained in the present Example had a higher treatment efficiency by approximately 25% than the results in (B) in FIG. 5 to provide better results.

### (Example 2)

Next, a photocatalyst-carrying mesh sheet was produced in the same manner as in Example 1 except that an expanded titanium mesh sheet was changed to a titanium wire mesh sheet in Example 1. The titanium wire mesh sheet of the present Example was obtained by subjecting a titanium wire having a diameter of 100 µm to plain weave, and had an opening ratio of 52%. The opening ratio of the photocatalyst-carrying mesh sheet obtained in the present Example was 52%. When the obtained photocatalyst-carrying mesh sheet was subjected to the same evaluation as that in Example 1, good results were obtained.

### (Example 3)

Next, the same as the expanded titanium mesh sheet used in Example 1 was subjected to a heat oxidation treatment for forming a titanium oxide film as follows. The heat oxidation treatment was performed in the atmosphere at 450 to 600°C for 2 hours. The titanium oxide film was formed by the heat oxidation treatment. In the present Example, a film having a thickness of 20 to 100 nm was formed.

Anatase type titanium oxide particles were supported in the same manner as in Example 1 to obtain a photocatalyst-carrying mesh sheet. The photocatalyst of the obtained photocatalyst-carrying mesh sheet provided a film having higher adhesion than that of Example 1.

The opening ratio of the photocatalyst-carrying mesh sheet obtained in the present Example was 47%.

When the obtained photocatalyst-carrying mesh sheet was subjected to the same evaluation as that in Example 1, the same good results as those in Example 1 were obtained.

### Industrial Applicability

The present invention can be applied to the uses of an air purifier which purifies air in medical facilities, factories, houses, and offices, a water purifier which purifies water, and a mosquito eliminator equipped with an air purification function.

### Description of Symbols

S1,2:Photocatalyst-carrying mesh sheet
U1:Photocatalyst unit
2:Through hole
3:Titanium oxide film
4:Anatase type titanium oxide particles
5:Photocatalyst layer
8:Crack
9:Ultraviolet light source
10:Treatment chamber
11:Expanded titanium mesh sheet
12:Titanium wire mesh sheet

## Claims

1. A photocatalyst-carrying mesh sheet comprising:
a net-form titanium sheet having a periodic pattern;
a titanium oxide film formed on a surface of the net-form titanium sheet; and
an anatase type titanium oxide particle supported on the titanium oxide film.

2. The photocatalyst-carrying mesh sheet according to claim 1, wherein the photocatalyst-carrying mesh sheet has an opening ratio of 10 to 80%.

3. An air purifier comprising the photocatalyst-carrying mesh sheet according to claim 1 or 2.

4. A method for producing a photocatalyst-carrying mesh sheet, the method comprising the steps of:
periodically forming a plurality of flaws in a metal substrate made of titanium, and pulling the metal substrate to form an expanded titanium mesh sheet having a net-form and a periodic pattern;
subjecting the expanded titanium mesh sheet to an anode oxidation treatment and/or a heat treatment to form a titanium oxide film on a surface of the expanded titanium mesh sheet; and
supporting an anatase type titanium oxide particle on the titanium oxide film.

5. The method according to claim 4, wherein the expanded titanium mesh sheet is subjected to a roughening processing, and thereafter subjected to the anode oxidation treatment and/or the heat treatment.

6. The method according to claim 4 or 5, wherein the expanded titanium mesh sheet has an opening part having a length of 0.05 to 5 mm in a short width direction and a length of 0.1 to 10 mm in a long width direction.

7. The method according to any one of claims 4 to 6, wherein the plurality of flaws are formed by pressing or laser irradiation.

8. A method for producing a photocatalyst-carrying mesh sheet, the method comprising the steps of:
weaving a titanium wire into a titanium wire mesh sheet having a net-form and a periodic pattern;
subjecting the titanium wire mesh sheet to an anode oxidation treatment and/or a heat treatment to form a titanium oxide film on a surface of the titanium wire mesh sheet; and
supporting an anatase type titanium oxide particle on the titanium oxide film.

9. The method according to claim 8, wherein the titanium wire mesh sheet is selected from plain weave, twill weave, tatami weave, plain tatami weave, and twill tatami weave.
